# FASCICULE DE BREVET EUROPEEN

(11) **EP 1 037 597 B1**
(45) Date de publication et mention de la délivrance du brevet: **16.08.2001**
(21) Numéro de dépôt: 98956956.1
(22) Date de dépôt: 26.11.1998
(51) Int. Cl.: A61K 7/48

(54) **UTILISATION DE POLYMERES HYPERBRANCHES ET DE DENDRIMERES COMPORTANT UN GROUPEMENT PARTICULIER, EN TANT QU'AGENT FILMOGENE, LES COMPOSITIONS FILMOGENES LES COMPRENANT ET LEUR UTILISATION NOTAMMENT EN COSMETIQUE OU EN PHARMACIE**
Verwendung von hyperverzweigten Polymeren oder Dendrimeren mit besonderen Gruppen als filmbildendes Mittel, dieses Mittel enthaltende filmbildende Zusammensetzungen und deren Verwendung in der Kosmetik oder in der Pharmazie
USE OF HYPERBRANCHED POLYMERS AND DENDRIMERS COMPRISING A PARTICULAR GROUP AS FILM-FORMING AGENT, FILM-FORMING COMPOSITIONS COMPRISING SAME AND USE PARTICULARLY IN COSMETICS AND PHARMACEUTICS

(30) Priorité: 19.12.1997 FR 9716175
(43) Date de publication de la demande: 27.09.2000
(73) Titulaire: L'OREAL, 75008 Paris (FR)
(72) Inventeur: MAIGNAN, Jean, F-93290 Tremblay en France (FR); GENARD, Sylvie, F-75012 Paris (FR)
(74) Mandataire: Dodin, Catherine
(86) Numéro de dépôt international: FR9802537
(87) Numéro de publication internationale: WO9932076

(56) Documents cités:
- WO-A-97/14404
- DE-A- 1 595 016
- TORU TAKAGISHI ET ALL.: "Macromolecule-Small Molecule Interactions; Introduction of Additional Binding Sites in Polyethyleneimine by Disulfide Cross-linkages" BIOPOLYMERS, vol. 11, 1972, pages 483-491, XP002079404 US

## Description

La présente invention concerne l'utilisation de nouveaux composés permettant notamment l'obtention de films sur un support, et leur application notamment en cosmétique ou en pharmaceutique.

Les polymères hyperbranchés et les dendrimères sont bien connus dans l'art antérieur. Les polymères hyperbranchés sont des constructions moléculaires ayant une structure ramifiée, en général autour d'un coeur. Leur structure est en règle générale dépourvue de symétrie : les unités de base ou monomères ayant servi à la construction du polymère hyperbranché peuvent être de natures différentes et leur répartition est irrégulière. Les branches du polymère peuvent être de natures et de longueurs différentes. Le nombre d'unités de base, ou monomères, peut être différent suivant les différentes ramifications. Tout en étant asymétriques, les polymères hyperbranchés peuvent avoir : une structure extrêmement ramifiée, autour d'un coeur ; des couches ou générations successives de ramifications; une couche de chaînes terminales.
Les polymères hyperbranchés sont généralement issus de la polycondensation d'un ou plusieurs monomères ABx, A et B étant des groupements réactifs susceptibles de réagir ensemble, x étant un entier supérieur ou égal à 2, mais d'autres procédés de préparation peuvent être envisagés. Les polymères hyperbranchés se caractérisent par leur degré de polymérisation DP = 1-b, b étant le pourcentage de fonctionnalités, non terminales, de B qui n'ont pas réagi avec un groupement A. La condensation étant non systématique, au contraire de la synthèse de dendrimères, le degré de polymérisation est inférieur à 100%. On peut faire réagir un groupement terminal T sur le polymère hyperbranché pour obtenir une fonctionnalité particulière en extrémité de chaînes.
Plusieurs polymères hyperbranchés peuvent être associés entre eux, par une liaison covalente ou un autre type de liaison, par l'intermédiaire de leurs groupes terminaux. De tels polymères, dits pontés, entrent dans la définition des polymères hyperbranchés selon la présente invention.

Les dendrimères sont des polymères et oligomères hautement ramifiés ayant une structure chimique bien définie. En règle générale, les dendrimères comprennent un coeur, un nombre déterminé de générations de branches, ou fuseaux, et des groupes terminaux. Les générations de fuseaux sont constituées d'unités structurelles, qui sont identiques pour une même génération de fuseaux et qui peuvent être identiques ou différentes pour des générations de fuseaux différentes. Les générations de fuseaux s'étendent ràdialement en une progression géométrique à partir du coeur. Les groupes terminaux d'un dendrimère de la N^{ième} génération sont les groupes fonctionnels terminaux des fuseaux de la N^{ième} génération ou génération terminale.

La définition des dendrimères donnée ci-dessus inclut des molécules à ramifications symétriques ; elle inclut également des molécules à ramification non symétrique, comme par exemple les dendrimères dont les fuseaux sont des groupements lysine, dans lesquels le branchement d'une génération de fuseaux sur la précédente se fait sur les amines α et ε de la lysine, ce qui conduit à une différence dans la longueur des fuseaux des différentes ramifications.
Les polymères denses en étoiles, ou « dense star polymer », les polymères éclatés en étoile, ou « starburst polymer », les dendrimères en baguette, ou « rodshaped dendrimer », sont inclus dans la présente définition des dendrimères. Les molécules dénommées arborols et molécules en cascade entrent également dans la définition des dendrimères selon la présente invention.
Plusieurs dendrimères peuvent être associés entre eux, par une liaison covalente ou un autre type de liaison, par l'intermédiaire de leurs groupes terminaux pour donner des entités connues sous le nom de « dendrimères pontés », « agrégats de dendrimères » ou « bridged dendrimer ». De telles entités sont incluses dans la définition des dendrimères selon la présente invention.
Des dendrimères peuvent se présenter sous la forme d'un ensemble de molécules de même génération, ensembles dits monodisperses ; ils peuvent également se présenter sous la forme d'ensembles de générations différentes, dits polydisperses. La définition des dendrimères selon la présente invention inclut des ensembles monodisperses aussi bien que polydisperses de dendrimères.

On connaît notamment, par la demande de brevet français FR97-04085 au nom de la demanderesse, de nouveaux polymères choisis parmi les polymères hyperbranchés et les dendrimères, et comportant des groupements fonctionnels répondant à la formule suivante : 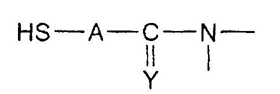 dans laquelle
* Y représente l'atome d'oxygène ou un groupement NH,
* A représente un groupe alcane di-yle en C₁-C₁₂, linéaire, ramifié ou cyclique, saturé ou insaturé; ce groupe étant éventuellement interrompu par un ou plusieurs hétéroatomes et/ou substitué par une fonction choisie parmi amino, acylamino, acide carboxylique et ester.
   Ces polymères trouvent notamment une application en cosmétique et dermatologie comme agent antioxydant ou agent réducteur.

WO97/14404 divulgue l'utilisation des dendromères comme agents adoucissants dans des compositions cosmétiques. Les dendrimères sont différent quant à la mode de liaison entre les parties des molecules.

Or, la demanderesse a constaté avec étonnement que lesdits polymères pouvaient également être utilisés pour permettre la préparation de compositions, notamment cosmétiques ou pharmaceutiques, permettant l'obtention d'un film lorsqu'elles étaient déposées sur un support.

Ainsi, la présente invention a pour objet l'utilisation d'au moins un composé choisi parmi les polymères hyperbranchés et les dendrimères, comportant au moins un groupement de formule : 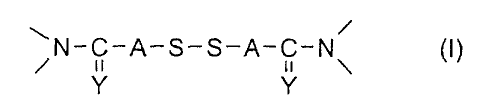 dans laquelle :
* Y représente l'atome d'oxygène ou un groupe NH, et
* A représente un groupe alcane di-yle en C₁-C₁₂, linéaire, ramifié ou cyclique, saturé ou insaturé; ce groupe étant éventuellement interrompu par un ou plusieurs hétéroatomes et/ou substitué par une fonction choisie parmi :
   - amino (-NH₂);
   - acylamino (-NH-CO-R) dans lequel R représente un groupement alkyle en C₁-C₁₀ linéaire, ramifié ou cyclique, saturé ou insaturé,
   - acide carboxylique (-COOH),
   - ester (-COOR) dans lequel R représente un groupement alkyle en C₁-C₁₀ linéaire, ramifié ou cyclique, saturé ou insaturé,
      en tant qu'agent filmogène.

L'invention a aussi pour objet une composition filmogène comprenant au moins un composé choisi parmi les polymères hyperbranchés et les dendrimères, comportant au moins un groupement de formule (I), ladite composition étant susceptible d'être obtenue par oxydation d'une composition comprenant au moins un polymère choisi parmi les polymères hyperbranchés et les dendrimères comportant au moins un groupement de formule (II).

L'invention a également pour objet l'utilisation d'au moins un polymère choisi parmi les polymères hyperbranchés et les dendrimères comportant au moins un groupement de formule (II) pour la préparation d'une composition filmogène comprenant au moins un composé choisi parmi les polymères hyperbranchés et les dendrimères comportant au moins un groupement de formule (I), ou d'une composition telle que ci-dessus définie.

L'invention a encore pour objet un procédé d'obtention d'un film sur un support, dans lequel on applique sur ledit support une composition telle que ci-dessus définie.

L'invention a également pour objet un procédé d'obtention d'un film sur un support, dans lequel on applique sur ledit support une composition comprenant un polymère choisi parmi les polymères hyperbranchés et tes dendrimères comportant au moins un groupement de formule (II), et l'on oxyde ladite composition pendant ou après son application sur ledit support.

On connaît dans l'art antérieur, un certain nombre d'agents filmogènes permettant l'obtention de films. En particulier, on peut citer les polymères filmogènes qui peuvent être des polymères radicalaires ou des polycondensats, parmi lesquels on peut citer les polymères acryliques ou les polyuréthannes. De tels polymères sont notamment utilisés dans le domaine capillaire, par exemple dans les laques, ou dans le domaine du maquillage, par exemple dans les mascaras ou les vernis à ongles en milieu solvant organique.

Toutefois, ces agents filmogènes présentent certains inconvénients. En particulier, ces agents filmogènes filmifient dès qu'ils sont déposés sur le support.
Au contraire, les composés selon l'invention ne filmifient qu'en milieu oxydant. Ainsi, il est possible d'appliquer en couche mince, une solution aqueuse ou hydroalcoolique, par exemple, de thiols (II) sur un support, puis, au moment choisi, d'oxyder chimiquement ou de laisser oxyder librement, notamment à l'air libre, lesdits thiols en composés (I), composés qui formeront, en séchant, sur le support, un film très adhérent et lavable à l'eau.

Les composés selon l'invention permettent l'obtention de films semblables à ceux obtenus avec des polymères filmogènes de l'art antérieur.
Selon les conditions d'oxydation, la qualité des films obtenus peut varier : films collants ou non, films brillants ou mats, etc.
Ainsi, en fonction des conditions d'oxydation, on peut aisément obtenir des films brillants, adhérant bien sur le support et sont non collants; de tels films, lorsqu'ils comprennent un pigment ou un colorant, ne tachent pas le support sur lequel ils sont déposés, car ils ne dégorgent pas.

Les composés susceptibles d'être utilisés dans le cadre de l'invention sont choisis parmi les polymères hyperbranchés et les dendrimères, et comportent au moins un groupement de formule (I): 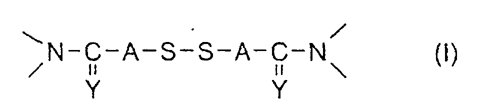 dans laquelle :
* Y représente O ou NH,
* A représente un groupe alcane di-yle en C₁-C₁₂, linéaire, ramifié ou cyclique, saturé ou insaturé; ce groupe étant éventuellement interrompu par un ou plusieurs hétéroatomes et/ou substitué par une fonction choisie parmi :
   - amino (-NH₂) éventuellement sous forme d'un sel d'un acide minéral ou organique,
   - acylamino (-NH-COR) dans lequel R représente un groupement alkyle en C₁-C₁₀ linéaire, ramifié ou cyclique, saturé ou insaturé,
   - acide carboxylique (-COOH),
   - ester (-COOR) dans lequel R représente un groupement alkyle en C₁-C₁₀ linéaire, ramifié ou cyclique, saturé ou insaturé.

De préférence, le composé selon l'invention est choisi parmi les polymères hyperbranchés, et notamment la polyéthylèneimine, comportant au moins un groupement de formule (I).
De préférence, Y représente l'atome d'oxygène.
De préférence, les hétéroatomes sont choisis parmi l'oxygène ou l'azote (O et N).
De préférence, A est un groupement méthylène, éthylène, propylène, méthylpropylène, éthylpropylène, tétraméthylène, pentaméthylène, hexaméthylène, phènylène, phényl di-yle.
Avantageusement, A représente un radical répondant à l'une des formules (a) à (d) suivantes :
(a) -CHR¹-CHR²-CHR³-
(b) -CHR'¹-CHR'²-CHR'³- CHR'⁴-
(c) 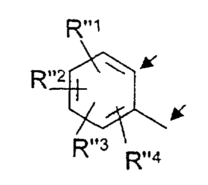
(d) -(CHR'''¹)ₖ-(CHR'''²)-CH(CO₂H)-NH-
dans lesquelles
* R¹, R², R³, R'¹, R'², R'³ et R'⁴, R'''¹, R'''², identiques ou différents représentent: l'atome d'hydrogène; un radical alkyle en C₁-C₆, linéaire, ramifié ou cyclique, saturé ou insaturé; un radical amino (-NH₂); un radical acide carboxylique (-COOH); un radical alkylamino en C₁-C₁₀; un radical acylamino en C₁-C₁₀.
* R"¹, R"², R"³ et R"⁴, identiques ou différents représentent : l'atome d'hydrogène; un radical alkyle en C₁-C₄, linéaire ou ramifié, saturé ou insaturé; les flèches indiquant les positions des substitutions;
* k est un entier, préférentiellement 0 ou 1.

Préférentiellement, A est choisi parmi les groupes suivants :
-CH₂-CH(CO₂H)-NH- ; -(CH₂)₂-(CH₃CONH)CH- ; -(CH₂)₃- et -CH₂-CH(NH-CO-CH₃)-

Les composés ci-dessus définis peuvent notamment être obtenus par oxydation des polymères décrits dans la demande FR97-04085 dont le contenu est incorporé par référence, et qui sont choisis parmi les polymères hyperbranchés et les dendrimères, comportant des groupements fonctionnels répondant à la formule (II) 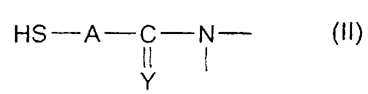 dans laquelle :
* Y représente O ou NH,
* A représente un groupe alcane di-yle en C₁-C₁₂, linéaire, ramifié ou cyclique, saturé ou insaturé; ce groupe étant éventuellement interrompu par un ou plusieurs hétéroatomes et/ou substitué par une fonction choisie parmi amino, acylamino, acide carboxylique et ester.
   L'oxydation peut être effectuée par tout moyen connu, par exemple à l'air ou à l'aide d'un oxydant usuel tel que le peroxyde d'hydrogène,

L'étape d'oxydation permet la formation de ponts disulfures, intramoléculaires et intermoléculaires, à partir des fonctions thiols, selon le schéma ci-dessous:

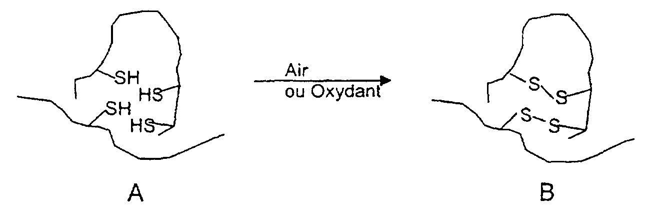

La formation de ponts disulfures entraîne une 'pseudo-réticulation' des composés A de départ, qui se traduit par la formation de composés B qui permettent l'obtention de films lorsqu'ils sont déposés sur un support.

L'étape d'oxydation est de préférence effectuée en présence d'eau, par exemple en milieu aqueux ou hydroalcoolique.

Après application sur un support, on peut ainsi obtenir des films ne renfermant qu'un ou plusieurs composés B selon l'invention et éventuellement des composés de départ A n'ayant pas réagi, lorsque l'oxydation n'est conduite que partiellement.

On a également constaté qu'il était possible d'incorporer dans le milieu aqueux ou hydroalcoolique, avant oxydation, des additifs hydrosolubles ou non hydrosolubles, tout en conservant la possibilité d'obtenir un film adéquat.
Parmi les additifs susceptibles d'être incorporés, on peut citer les colorants hydrosolubles tels que la Rhodamine; des actifs cosmétiques ou pharmaceutiques hydrosolubles; des produits non hydrosolubles et présentant notamment des propriétés optiques telles que phosphorescence ou fluorescence; des pigments; des charges; des filtres solaires; des actifs cosmétiques ou pharmaceutiques non hydrosolubles.
On a de plus constaté que les additifs solides, particules de pigments ou de charges par exemple, étaient parfaitement dispersés au sein du film, de manière homogène; ils sont parfaitement emprisonnés dans la structure du film. Il n'y a pas de relargage des particules, et les films ne sont pas tachants.

On sait que les propriétés des films obtenus dépendent des conditions d'oxydation, en particulier de la concentration en polymère thiolé de départ, du nombre de fonctions thiols dudit polymère thiolé, de la masse molaire dudit polymère et/ou du pH du milieu aqueux/hydroalcoolique avant oxydation.

Ainsi, les films peuvent être réalisés directement à partir d'une solution aqueuse ou d'une solution hydroalcoolique de polymère thiolé. Lors du séchage, il y aura oxydation des fonctions thiols en disulfures. On a constaté que dans ce cas, les films obtenus peuvent être visuellement irréguliers et peuvent demeurer collants.
Lorsque l'on oxyde chimiquement une solution aqueuse ou hydroalcoolique de polymère thiolé et que l'on réalise un film à partir de cette solution oxydée liquide, on obtient généralement après séchage un film très régulier, à surface très régulière, brillant, transparent, incolore, très adhérent et qui peut être cassant lorsqu'on veut le décoller. Ces films ne sont pas résistants à l'eau.

On peut donc ainsi obtenir une composition susceptible de former des films, et qui peut être utilisée telle quelle, en tant que composition cosmétique ou pharmaceutique, ou incorporée à une composition notamment cosmétique ou pharmaceutique, qui peut alors comprendre un milieu cosmétiquement ou pharmaceutiquement acceptable.

Ladite composition cosmétique ou pharmaceutique peut se présenter sous toutes les formes appropriées pour une application topique, notamment sous forme de gels aqueux ou hydroalcooliques; sous forme d'émulsions eau-dans-huile, huile-dans-eau ou multiple, de consistance liquide plus ou moins épaissie, telles que lait ou crème; de sprays ou de mousse aérosol; de sticks ou bâtons; de solutions ou dispersions liquides.

L'homme du métier sait préparer ces compositions selon les méthodes usuelles, sur la base de ses connaissances générales.
En particulier, ces compositions peuvent contenir des adjuvants habituellement utilisés dans les domaines cosmétique ou pharmaceutique, tels que des huiles, cires ou autres corps gras usuels; des tensioactifs; des agents hydratants; des émollients; des filtres solaires; des actifs hydrophiles ou lipophiles comme des céramides; des agents anti-radicaux libres; des polymères ; des protéines; des bactéricides ; des séquestrants ; des antipelliculaires ; des antioxydants; des conservateurs ; des agents alcalinisants ou acidifiants; des parfums; des charges; des matières colorantes; des actifs cosmétiques ou pharmaceutiques. Les quantités de ces différents adjuvants sont celles classiquement utilisées dans les domaines considérés et sont aisément déterminables par l'homme du métier.
Bien entendu l'homme du métier veillera à choisir ce ou ces éventuels adjuvants complémentaires, et/ou leur quantité, de manière telle que les propriétés avantageuses de la composition selon l'invention ne soient pas, ou substantiellement pas, altérées par l'adjonction envisagée.

Les compositions selon l'invention sont filmogènes et peuvent donc être utilisées pour former un film sur un support, notamment choisi parmi la peau, les muqueuses, les semi-muqueuses, les ongles, les cheveux de l'être humain.

Les compositions selon l'invention sont par exemple des lotions, des laits ou des crèmes pour le soin de la peau ou des cheveux; des crèmes, des lotions ou des laits démaquillants; des bases de fond de teint; des lotions, des laits ou des crèmes antisolaires ou après-soleil; des lotions, des laits ou des crèmes de bronzage artificiel; des crèmes ou des mousses de rasage; des lotions après rasage; des compositions d'hygiène corporelle telles que des sticks ou des crèmes déodorants; des shampooings; des produits capillaires pour le maintien de la coiffure ou la mise en forme des cheveux tels que des gels de coiffage; des produits de coloration des cheveux; des rouges à lèvres; des mascaras ou eye-liners éventuellement traitants; des vernis à ongles ou des soins des ongles.

L'invention est illustrée plus en détail dans les exemples suivants. Les exemples 1 à 3 décrivent la préparation des composés de départ de formule (II).

Les exemples 4 à 6 décrivent la préparation de films selon l'invention.

### Exemple 1: Polymère branché polyéthylèneimine de poids moléculaire moyen PM=25000 possédant 50 fonctions SH en moyenne par motif

A 12,28 grammes de solution aqueuse à 56% de polyéthylèneimine de poids moléculaire moyen PM=25000 commercialisée par la société BASF sous la dénomination LUPASOL HF, on ajoute à température ambiante 12,28 g d'eau puis, lorsque le milieu est redevenu homogène, 1,2 ml de γ-thiobutyrolactone (soit 50 équivalents molaires calculés par rapport au polymère) sous atmosphère inerte à température ambiante. Le milieu, initialement hétérogène, devient rapidement homogène (environ 30 minutes). Après 24 heures sous agitation, on ne détecte plus de γ-thiobutyrolactone dans le milieu.
On dispose ainsi d'une solution aqueuse de poly(éthylèneimine) thiolée de masse molaire 30108 renfermant en moyenne 50 fonctions SH par chaîne de polymère.
On dilue 19,60 g de cette solution par de l'eau qsp 25 ml. On dispose ainsi d'une solution aqueuse à 25 g/100 ml en polymère thiolé soit 0,415 mol/l en thiol et 8,30 mmol/l en polymère thiolé. Le pH de cette solution est de 10,65.

### Exemple 2 : Polymère branché polyéthylèneimine de poids moléculaire moyen PM=25000 possédant 125 fonctions SH en moyenne par motif

A 22,43 grammes de solution aqueuse à 56% de polyéthylèneimine de poids moléculaire moyen PM=25000 commercialisée par la société BASF sous la dénomination LUPASOL HF, on ajoute à température ambiante 22,43 g d'eau puis 5,44 ml de γ-thiobutyrolactone (soit 125 équivalents molaires calculés par rapport au polymère) sous atmosphère inerte à température ambiante. Le milieu, initialement hétérogène, devient rapidement homogène (environ 30 minutes). Après 24 heures sous agitation, on ne détecte plus de γ-thiobutyrolactone dans le milieu. On dispose ainsi d'une solution aqueuse de poly(éthyièneimine) thiolée de masse molaire 37770, renfermant en moyenne 125 fonctions SH par chaîne de polymère.
On dilue 32,080g de cette solution par de l'eau qsp 50 ml. On dispose ainsi d'une solution aqueuse à 25 g/100 ml en polymère thiolé soit 0,827 mol/l en thiol et 6,619 mmol/l en polymère thiolé. Le pH de cette solution est de 9,96.

### Exemple 3 : Polymère branché polyéthylèneimine de poids moléculaire moyen PM=2000 possédant 11,09 fonctions SH en moyenne par motif

A 50 grammes de solution aqueuse à 50% de polyéthylèneimine de poids moléculaire moyen PM=2000 commercialisée par la société BASF sous la dénomination LUPASOL G35, on ajoute à température ambiante 12 ml de γ-thiobutyrolactone (soit 11,09 équivalents molaires calculés par rapport au polymère) sous atmosphère inerte à température ambiante. Le milieu, initialement hétérogène, devient rapidement homogène (environ 30 minutes). Après 16 heures sous agitation, on ne détecte plus de γ-thiobutyrolactone dans le milieu. On dispose ainsi d'une solution aqueuse de poly(éthylèneimine) thiolée de masse molaire 3132,95 renfermant en moyenne 11,09 fonctions SH par chaîne de polymère.

On dilue cette phase aqueuse par de l'eau qsp 100 ml. On dispose ainsi d'une solution aqueuse à 39,16 g/100 ml en polymère thiolé soit 1,386 mol/l en thiol. Le pH de cette solution est de 10,15.

### Exemple 4 : Préparation de films à partir de poly(éthylèneimine) thiolée selon l'exemple 1

### a) première série

A partir de la solution à 25 g/100 ml préparée dans l'exemple 1, on réalise diverses solutions par dilution à l'eau et/ou acidification par une solution aqueuse d'acide chlorhydrique.
Leurs caractéristiques sont les suivantes:

| Solution/ film | pH | Concentration en polymère thiolé | Concentration en thiol (mol/l) |
|---|---|---|---|
| 1 | 10,65 | 8,303 mM 25 g/100 ml | 0,415 |
| 2 | 8,9 | 6,323 mM 19,05 g/100 ml | 0,316 |
| 3 | 7,9 | 5,641 mM 16.98 g/100 ml | 0,282 |
| 4 | 6,9 | 5,289 mM 15.923 g/100 ml | 0,264 |
| 5 | 5,7 | 4,942 mM 14,881 g/100 ml | 0,247 |

On réalise les films sur des plaques de verre de 10 cm x 10 cm. Les dépôts sont réalisés à l'aide d'un filmographe Baker Règlable (BRAIVE Instruments) et ont une épaisseur de 150 microns.
Ils sont laissés à sécher à l'air ambiant, à 20°C, sur une surface plane. On laisse donc les dépôts s'oxyder à l'air.

### b) deuxième série

A partir de la solution à 25 g/100 ml préparée dans l'exemple 1, on réalise également deux autres films pour lesquels le polymère est totalement oxydé par une solution aqueuse de H₂O₂ à 6%. La solution oxydée liquide est utilisée pour réaliser des films à partir de dépôts de 150 microns d'épaisseur sur des plaques de verre de 10 cm x 10 cm, à l'aide d'un filmographe Baker Règlable (BRAIVE Instruments). Ils sont laissés à sécher à l'air ambiant, à 20°C, sur une surface plane.

### Film 6 :

On prépare une solution aqueuse de polymère oxydé à partir de 1 volume de solution de polymère thiol préparé selon l'exemple 1 à 19,05 g/100 g (pH 8,96) dilué par 1 volume d'eau et oxydé par 1 équivalent d'eau oxygénée à 6% (le milieu reste liquide après oxydation des thiols en disulfure).
On réalise le film à partir de cette solution aqueuse.

### Film 7 :

On prépare une solution aqueuse de polymère oxydé à partir de 1 volume de solution de polymère thiol préparé selon l'exemple 1 à 15,92 g/100 ml (pH 6,97) dilué par 0,2 volume d'eau et oxydé par 1 équivalent d'eau oxygénée à 6%. On sait, par des essais préliminaires, que le milieu reste liquide quelques instants après ajout de l'oxydant, avant de gélifier.
On réalise le film à partir de cette solution aqueuse avant sa gélification.

### c) résultats des deux séries

On obtient 7 films dont les caractéristiques sont les suivantes, après 24 heures de séchage :
- film 1 : collant, rétracté, non uniforme en surface
- films 2 à 5 : collants, non uniformes en surface (présence de bulles), qui n'évoluent pas même après plusieurs jours de séchage
- film 6 : collant, uniforme en surface. Ce film devient sec et non collant après 48 heures de séchage. Il est alors incolore, brillant, transparent et très adhérent
- film 7 : sec, non collant, brillant, surface très lisse, transparent, incolore et très adhérent.
   Les films 1 à 7 sont lavables à l'eau.

### Exemple 5 : Préparation de films à partir de poly(éthylèneimine) thiolée selon l'exemple 2

### a) première série

A partir de la solution à 25 g/100 ml préparée dans l'exemple 2, on réalise diverses solutions par dilution à l'eau et/ou acidification par une solution aqueuse d'acide chlorhydrique.
Leurs caractéristiques sont les suivantes:

| Solution/ film | pH | Concentration en polymère thiolé | Concentration en thiol (mol/l) |
|---|---|---|---|
| 8 | 8,9 | 10,52 mM 2,78 g/100 ml | 0,348 |
| 9 | 7,9 | 3,46 mM 13,06 9/100 ml | 0,432 |
| 10 | 5,0 | 4,583 mM 17,313 g/100 ml | 0,573 |

On réalise les films sur des plaques de verre de 10 cm x 10 cm. Les dépôts sont réalisés à l'aide d'un filmographe Baker Réglable (BRAIVE Instruments) et ont une épaisseur de 250 microns.
Ils sont laissés à sécher à l'air ambiant, à 20°C, sur une surface plane. On laisse donc les dépôts s'oxyder à l'air.

### b) deuxième série

On réalise également quatre autres films pour lesquels le polymère est totalement oxydé par 1 équivalent d'une solution aqueuse de H₂O₂ à 6%.
La solution oxydée encore liquide, avant son éventuelle gélification, est utilisée pour réaliser des films à partir de dépôts de 250 microns d'épaisseur réalisés sur des plaques de verre de 10 cm x 10 cm, à l'aide d'un filmographe Baker Règlable (BRAIVE Instruments).
Ils sont laissés à sécher à l'air ambiant, à 20°C, sur une surface plane.

| Solution/ film | pH avant dilution | Concentration en polymère thiolé | Concentration en thiol (mol/l) |
|---|---|---|---|
| 11 | 7,9 | 2,36 mM 8,905 g/100 ml | 0,295 |
| 12 | 6,9 | 3,06 mM 11,56 g/100 ml | 0,382 |
| 13 | 5,8 | 3,96 mM 14,97 g/100 ml | 0,495 |
| 14 | 5,0 | 4,58 mM 17,31 g/100 ml | 0,573 |

### c) résultats des deux séries

On obtient 7 films dont les caractéristiques sont les suivantes, après 24 heures de séchage :
- film 8 : sec, non collant, brillant, transparent, incolore et très adhérent.
- film 9 : collant, rétracté, non uniforme en surface (présence de bulles), qui n'évolue pas même après plusieurs jours de séchage
- film 10 : collant, un peu rétracté
- films 11 à 14 : secs, non collants, brillants, à surface très lisse, transparents, incolores et très adhérents.
   Les films 8 à 14 sont lavables à l'eau.

### Exemple 6 : Préparation de films à partir de poly(éthylèneimine) thiolée selon l'exemple 3

### a) première série

A partir de la solution à 39,16 g/100 ml préparée dans l'exemple 3, on réalise une solution à pH 7, par acidification par une solution aqueuse d'acide chlorhydrique. Ses caractéristiques sont les suivantes:

| Solution/ film | pH | Concentration en polymère thiolé | Concentration en thiol (mol/l) |
|---|---|---|---|
| 15 | 7 | 82,668 mM 25,8994 g/100 ml | 0,9168 |

A partir de cette solution, on prépare deux échantillons aqueux ou hydroalcoolique (eau + éthanol absolu) renfermant :
- produit A : pigment coloré bleu (FD&C Blue n°1 Aluminium Lake, Cl 42090:2)
- produit B : pigment coloré rouge (D&C Red n°7, Calcium Lake) selon le tableau ci-dessous :

| Solution | Préparation | Concentration en polymère thiolé | Concentration en thiol (mol/l) | % de pigment * |
|---|---|---|---|---|
| 16 | 1 volume de sol.15 + 0,8 volume d'eau | 45,927 mM 14,388 g/100 ml | 0,5093 | 2% produit A |
| 17 | 1 volume de sol.15 + 0,7 volume EtOH | 48,628 mM 15,235 g/100 ml | 0,5393 | 1% produit B |

| | | | | |
|---|---|---|---|---|
| * calculé en poids par rapport au poids de polymère thiolé | | | | |

D'après des essais préliminaires, on sait que les solutions 16 et 17, après ajout de l'oxydant (1 équivalent de solution aqueuse de H₂O₂ à 6%), restent liquides quelques instant avant de gélifier.
On réalise les films à partir des solutions de polymères en cours d'oxydation, avant la gélification, c'est-à-dire pendant la réticulation (juste après ajout de 1 équivalent de solution aqueuse de H₂O₂ à 6%, l'oxydant étant ajouté sous agitation au vortex).

On réalise les films sur des plaques de verre de 10 cm x 10 cm. Les dépôts sont réalisés à l'aide d'un filmographe Baker Règlable (BRAIVE Instruments) et ont une épaisseur de 200 microns.

Ils sont laissés à sécher à l'air ambiant, à 20°C, sur une surface plane.

Après séchage, on obtient deux films colorés : le film 16 est bleu pâle transparent et le film 17 est orange transparent.
Ces deux films ont une surface très régulière, sont d'aspect brillant et apparaissent uniformes à l'oeil nu. Ils ne sont pas tachants. On ne distingue pas de particules de pigments. Ces films sont très adhérents et sont lavables à l'eau.

### b) seconde série

A partir de la solution 15, on prépare deux échantillons aqueux ou hydroalcooliques (eau + éthanol absolu) renfermant de la Rhodamine B (RN = [81-88-9]) selon le tableau ci-dessous :

| Solution | Préparation | Concentration en polymère thiolé | Concentration en thiol (mol/l) | % de pigment * |
|---|---|---|---|---|
| 18 | 1 volume de sol.15 + 0,7 volume d'eau | 48,628 mM 15,235 g/100 ml | 0,5393 | 0,1% |
| 19 | 1 volume de sol.15 + 0,17 volume EtOH + 0,41 volume d'eau | 52,45 mM 16,434 g/100 ml | 0,5817 | 0,028% |

| | | | | |
|---|---|---|---|---|
| * calculé en poids par rapport au poids de polymère thiolé | | | | |

D'après des essais préliminaires, on sait que les solutions 18 et 19, après ajout de 1 équivalent de solution aqueuse de H₂O₂ à 6%, restent liquides quelques instant avant de gélifier.
On réalise les films à partir des solutions de polymères en cours d'oxydation, avant la gélification, c'est-à-dire pendant la réticulation (juste après ajout de 1 équivalent de solution aqueuse de H₂O₂ à 6%, l'oxydant étant ajouté sous agitation au vortex).
On réalise les films sur des plaques de verre de 10 cm x 10 cm. Les dépôts sont réalisés à l'aide d'un filmographe Baker Réglable (BRAIVE Instruments) et ont une épaisseur de 200 microns.
Ils sont laissés à sécher à l'air ambiant, à 20°C, sur une surface plane.

Après séchage, on obtient deux films colorés rose pâle transparents. Ces deux films ont une surface très régulière, sont d'aspect brillant et apparaissent uniformes à l'oeil nu. Ils ne sont pas tachants. Ces films sont très adhérents et sont lavables à l'eau.

## Revendications

1. Utilisation non-thérapeutique d'au moins un composé choisi parmi les polymères hyperbranchés et les dendrimères, comportant au moins un groupement de formule : 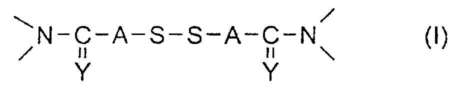 dans laquelle :
* Y représente l'atome d'oxygène ou un groupe NH, et
* A représente un groupe alcane di-yle en C₁-C₁₂, linéaire, ramifié ou cyclique, saturé ou insaturé; ce groupe étant éventuellement interrompu par un ou plusieurs hétéroatomes et/ou substitué par une fonction choisie parmi :
- amino (-NH₂);
- acylamino (-NH-CO-R) dans lequel R représente un groupement alkyle en C₁-C₁₀ linéaire, ramifié ou cyclique, saturé ou insaturé,
- acide carboxylique (-COOH),
- ester (-COOR) dans lequel R représente un groupement alkyle en C₁-C₁₀ linéaire, ramifié ou cyclique, saturé ou insaturé,
en tant qu'agent filmogène.

2. Utilisation selon la revendication 1, dans laquelle le composé est choisi parmi les polymères hyperbranchés, et notamment la polyéthylèneimine, comportant au moins un groupement de formule (I).

3. Utilisation selon l'une des revendications précédentes, dans laquelle Y représente l'atome d'oxygène.

4. Utilisation selon l'une des revendications précédentes, dans laquelle A représente un radical répondant à l'une des formules (a) à (d) suivantes :
(a) -CHR¹-CHR²-CHR³-
(b) -CHR'¹-CHR'²-CHR'³- CHR'⁴-
(c) 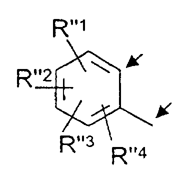
(d) -(CHR"'¹)ₖ-(CHR"'²)-CH(CO₂H)-NH-
dans lesquelles
* R¹, R², R³, R'¹, R'², R'³ et R'⁴, R'''¹, R'''², identiques ou différents représentent : l'atome d'hydrogène; un radical alkyle en C₁-C₆, linéaire, ramifié ou cyclique, saturé ou insaturé; un radical amino (-NH₂); un radical acide carboxylique (-COOH); un radical alkylamino en C₁-C₁₀; un radical acylamino en C₁-C₁₀.
* R"¹, R"², R"³ et R"⁴, identiques ou différents représentent : l'atome d'hydrogène; un radical alkyle en C₁-C₄, linéaire ou ramifié, saturé ou insaturé; les flèches indiquant les positions des substitutions;
* k est un entier, préférentiellement 0 ou 1.

5. Utilisation selon l'une des revendications précédentes, dans laquelle A est choisi parmi les groupes suivants :
-CH₂-CH(CO₂H)-NH- ; -(CH₂)₂-(CH₃CONH)CH- ; -(CH₂)₃- et -CH₂-CH(NH-CO-CH₃)-

6. Utilisation selon l'une des revendications précédentes, dans une composition cosmétique ou pharmaceutique qui comprend en outre un milieu cosmétiquement ou pharmaceutiquement acceptable.

7. Composition fiimogène comprenant au moins un composé choisi parmi les polymères hyperbranchés et les dendrimères, comportant au moins un groupement de formule (I), tel que défini dans l'une des revendications 1 à 5.

8. Composition selon la revendication 7, susceptible d'être obtenue par oxydation d'une composition comprenant au moins un polymère choisi parmi les polymères hyperbranchés et les dendrimères comportant au moins un groupement de formule (II) : 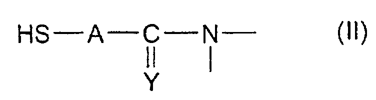 dans laquelle :
* Y représente l'atome d'oxygène ou un groupe NH, et
* A représente un groupe alcane di-yle en C₁-C₁₂, linéaire, ramifié ou cyclique, saturé ou insaturé; ce groupe étant éventuellement interrompu par un ou plusieurs hétéroatomes et/ou substitué par une fonction choisie parmi :
- amino (-NH₂);
- acylamino (-NH-COR) dans lequel R représente un groupement alkyle en C₁-C₁₀ linéaire, ramifié ou cyclique, saturé ou insaturé,
- acide carboxylique (-COOH),
- ester (-COOR) dans lequel R représente un groupement alkyle en C₁-C₁₀ linéaire, ramifié ou cyclique, saturé ou insaturé.

9. Composition selon l'une des revendications 7 à 8, dans laquelle l'oxydation est effectuée à l'air ou à l'aide d'un oxydant usuel tel que le peroxyde d'hydrogène.

10. Composition selon l'une des revendications 7 à 9, dans laquelle l'oxydation est effectuée en présence d'eau, par exemple en milieu aqueux ou hydroalcoolique.

11. Composition selon la revendication 10, dans laquelle on incorpore dans le milieu aqueux ou hydroalcoolique, avant oxydation, des additifs hydrosolubles ou non hydrosolubles.

12. Composition selon la revendication 11, dans laquelle lesdits additifs sont choisis parmi les colorants hydrosolubles tels que la Rhodamine; des actifs cosmétiques ou pharmaceutiques hydrosolubles; des produits non hydrosolubles et présentant notamment des propriétés optiques telles que phosphorescence ou fluorescence; des pigments; des charges; des filtres solaires; des actifs cosmétiques ou pharmaceutiques non hydrosolubles.

13. Composition selon l'une des revendications 7 à 12, se présentant sous la forme d'une composition cosmétique ou pharmaceutique, comprenant en outre un milieu cosmétiquement ou pharmaceutiquement acceptable.

14. Composition selon la revendication 13, se présentant sous la forme de lotions, de laits ou de crèmes pour le soin de la peau ou des cheveux; de crèmes, de lotions ou de laits démaquillants; de bases de fond de teint; de lotions, de laits ou de crèmes antisolaires ou après-soleil; de lotions, de laits ou de crèmes de bronzage artificiel; de crèmes ou de mousses de rasage; de lotions après rasage; de compositions d'hygiène corporelle telles que des sticks ou des crèmes déodorants; de shampooings; de produits capillaires pour le maintien de la coiffure ou la mise en forme des cheveux tels que des gels de coiffage; de produits de coloration des cheveux; de rouges à lèvres; de mascaras ou eye-liners éventuellement traitants; de vernis à ongles ou de soins des ongles.

15. Utilisation d'au moins un polymère choisi parmi les polymères hyperbranchés et les dendrimères comportant au moins un groupement de formule (II) : 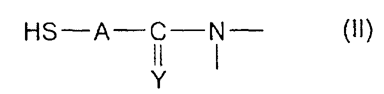 dans laquelle Y et A ont les significations définies ci-dessus, pour la préparation d'une composition filmogène comprenant au moins un composé choisi parmi les polymères hyperbranchés et les dendrimères comportant au moins un groupement de formule (I), ou d'une composition selon l'une des revendications 7 à 14.

16. Procédé d'obtention d'un film sur un support, dans lequel on applique sur ledit support une composition selon l'une des revendications 7 à 14.

17. Procédé d'obtention d'un film sur un support, dans lequel :
- on applique sur ledit support une composition comprenant un polymère choisi parmi les polymères hyperbranchés et les dendrimères comportant au moins un groupement de formule (II), et
- l'on oxyde ladite composition pendant ou après son application sur ledit support.

18. Procédé selon l'une des revendications 16 à 17, dans lequel le support est choisi parmi la peau, les muqueuses, les semi-muqueuses, les ongles, les cheveux.

## Patentansprüche

1. Nicht therapeutische Verwendung mindestens einer Verbindung als Filmbildner, die unter den hochverzweigten Polymeren und den Dendrimeren ausgewählt ist, die mindestens eine Gruppe der folgenden Formel aufweisen: 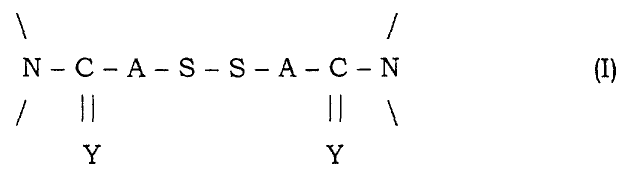 worin bedeuten:
- Y Sauerstoff oder die Gruppe NH, und
- A eine geradkettige, verzweigte oder cyclische, gesättigte oder ungesättigte C₁₋₁₂-Alkandiylgruppe, wobei diese Gruppe gegebenenfalls durch ein oder mehrere Heteroatome unterbrochen und/oder mit einer der folgenden Gruppen substituiert ist:
- Amino (-NH₂);
- Acylamino (-NH-CO-R), wobei R eine geradkettige, verzweigte oder cyclische, gesättigte oder ungesättigte C₁₋₁₀-Alkylgruppe bedeutet,
- Carboxy (-COOH),
- Ester (-COOR), wobei R eine geradkettige, verzweigte oder cyclische, gesättigte oder ungesättigte C₁₋₁₀-Alkylgruppe bedeutet.

2. Verwendung nach Anspruch 1, wobei die Verbindung unter den hochverzweigten Polymeren und insbesondere Polyethylenimin ausgewählt ist, die mindestens eine Gruppe der Formel (I) enthalten.

3. Verwendung nach einem der vorhergehenden Ansprüche, wobei Y Sauerstoff bedeutet.

4. Verwendung nach einem der vorhergehenden Ansprüche, wobei A eine Gruppe bedeutet, die einer der folgenden Formeln (a) bis (d) entspricht:
(a) -CHR¹-CHR²- CHR³-
(b) -CHR'¹-CHR'²-CHR'³- CHR'⁴-
(c) 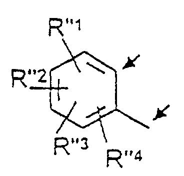
(d) -(CHR"'¹)ₖ-(CHR"'²)-CH(CO₂H)-NH-
worin bedeuten: -
- die Gruppen R¹, R², R³, R'¹, R'², R'³, R'⁴, R"'¹ und R"'², die identisch oder voneinander verschieden sind, Wasserstoff, eine geradkettige, verzweigte oder cyclische, gesättigte oder ungesättigte C₁₋₆-Alkylgruppe, Amino (-NH₂), Carboxy (-COOH), C₁₋₁₀-Alkylamino oder C₁₋₁₀-Acylamino,
- die Gruppen R"¹, R"², R"³ und R"⁴, die identisch oder voneinander verschieden sind, Wasserstoff oder eine geradkettige oder verzweigte, gesättigte oder ungesättigte C₁₋₄-Alkylgruppe, wobei die Pfeile die Substitutionsstellungen angeben,
- k eine ganze Zahl, vorzugsweise 0 oder 1.

5. Verwendung nach einem der vorhergehenden Ansprüche, wobei A unter den folgenden Gruppen ausgewählt ist:
-CH₂-CH(CO₂H)-NH-; -(CH₂)₂-(CH₃CONH)CH-; -(CH₂)₃-; und -CH₂-CH(NH-CO-CH₃)-.

6. Verwendung nach einem der vorhergehenden Ansprüche in einer kosmetischen oder pharmazeutischen Zusammensetzung, die ferner ein kosmetisch oder pharmazeutisch akzeptables Medium enthält.

7. Filmbildende Zusammensetzung , die mindestens eine in einem der Ansprüche 1 bis 5 definierte Verbindung enthält, die unter den hochverzweigten Polymeren und Dendrimeren ausgewählt ist, die mindestens eine Gruppe der Formel (I) aufweisen.

8. Zusammensetzung nach Anspruch 7, die durch Oxidation einer Zusammensetzung hergestellt werden kann, die mindestens ein Polymer enthält, das unter den hochverzweigten Polymeren und Dendrimeren ausgewählt ist, die mindestens eine Gruppe der folgenden Formel (II) aufweisen: 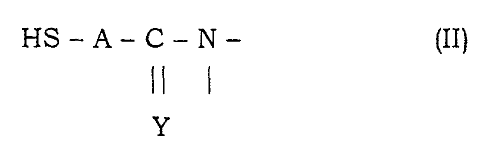 worin bedeuten:
- Y Sauerstoff oder die Gruppe NH, und
- A eine geradkettige, verzweigte oder cyclische, gesättigte oder ungesättigte C₁₋₁₂-Alkandiylgruppe, wobei diese Gruppe gegebenenfalls durch ein oder mehrere Heteroatome unterbrochen und/oder mit einer der folgenden Gruppen substituiert ist:
- Amino (-NH₂);
- Acylamino (-NH-CO-R), wobei R eine geradkettige, verzweigte oder cyclische, gesättigte oder ungesättigte C₁₋₁₀-Alkylgruppe bedeutet,
- Carboxy (-COOH),
- Ester (-COOR), wobei R eine geradkettige, verzweigte oder cyclische, gesättigte oder ungesättigte C₁₋₁₀-Alkylgruppe bedeutet.

9. Zusammensetzung nach einem der Ansprüche 7 bis 8, wobei die Oxidation an Luft oder mit einem herkömmlichen Oxidationsmittel, wie Wasserstoffperoxid, durchgeführt wird.

10. Zusammensetzung nach einem der Ansprüche 7 bis 9, wobei die Oxidation in Gegenwart von Wasser, beispielsweise in einem wäßrigen oder wäßrig-alkoholischen Medium durchgeführt wird.

11. Zusammensetzung nach Anspruch 10, wobei vor der Oxidation in das wäßrige oder wäßrig-alkoholische Medium wasserlösliche oder nicht wasserlösliche Zusatzstoffe eingearbeitet werden.

12. Zusammensetzung nach Anspruch 11, wobei die Zusatzstoffe ausgewählt sind unter: wasserlöslichen Farbstoffen, wie Rhodamin; wasserlöslichen kosmetischen oder pharmazeutischen Wirkstoffen; nicht wasserlöslichen Produkten, die insbesondere optische Eigenschaften aufweisen, wie Phosphoreszenz oder Fluoreszenz; Pigmenten; Füllstoffen; Sonnenschutzfiltern; und nicht wasserlöslichen kosmetischen oder pharmazeutischen Wirkstoffen.

13. Zusammensetzung nach einem der Ansprüche 7 bis 12, wobei sie in Form einer kosmetischen oder pharmazeutischen Zusammensetzung vorliegt, die ferner ein kosmetisch oder pharmazeutisch akzeptables Medium enthält.

14. Zusammensetzung nach Anspruch 13, wobei sie in Form von Lotionen, Milchen oder Cremes zur Pflege der Haut oder der Haare; Cremes, Lotionen oder Milchen zum Abschminken; Grundmassen für Make-up; Lotionen, Milchen oder Cremes zum Sonnenschutz oder entsprechenden After-sun-Produkten; Lotionen, Milchen oder Cremes zur Selbstbräunung; Cremes oder Schäumen für die Rasur; After-Shave-Lotionen; Zusammensetzungen für die Körperhygiene, beispielsweise Deostiften oder desodorierenden Cremes; Haarwaschmitteln; Produkten für das Haar zur Festigung oder Formgebung, wie Frisiergelen; Produkten zum Färben der Haare; Lippenstiften; Mascaras oder Eyelinern, die gegebenenfalls behandelnd wirken; und dekorativen oder pflegenden Nagellacken vorliegt.

15. Verwendung mindestens eines Polymers, das unter den hochverzweigten Polymeren und Dendrimeren ausgewählt ist, die mindestens eine Gruppe der folgenden Formel aufweisen: 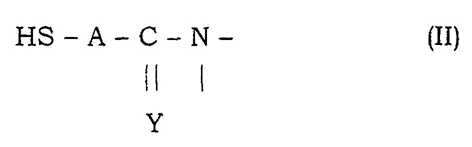 worin Y und A die oben angegebenen Bedeutungen aufweisen, zur Herstellung einer filmbildenden Zusammensetzung, die mindestens eine Verbindung enthält, die unter den hochverzweigten Polymeren und Dendrimeren ausgewählt ist, die mindestens eine Gruppe der Formel (I) aufweisen, oder einer Zusammensetzung nach einem der Ansprüche 7 bis 14.

16. Verfahren zur Herstellung eines Films auf einem Träger, wobei auf den Träger eine Zusammensetzung nach einem der Ansprüche 7 bis 14 aufgetragen wird.

17. Verfahren zur Herstellung eines Films auf einem Träger, wobei:
- auf den Träger eine Zusammensetzung aufgebracht wird, die ein Polymer enthält, das unter den hochverzweigten Polymeren und den Dendrimeren ausgewählt ist, die mindestens eine Gruppe der Formel (II) aufweisen, und
- die Zusammensetzung oxidiert wird, während sie auf den Träger aufgebracht wird oder nachdem sie auf den Träger aufgebracht wurde.

18. Verfahren nach einem der Ansprüche 16 bis 17, wobei der Träger unter der Haut, den Schleimhäuten, den Semischleimhäuten, den Nägeln und den Haaren ausgewählt ist.

## Claims

1. Non-therapeutic usa of at least one compound chosen from hyperbranched polymers and dendrimers, comprising at least one group of formula: 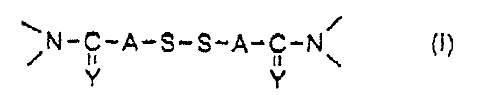 in which:
* Y represents an oxygen atom or an NH group, and
* A represents a linear, branched or cyclic, saturated or unsaturated C₁-C₁₂ alkanediyl group, this group optionally being interrupted with one or more hetero atoms and/or substituted with a function chosen from:
- amino (-NH₂),
- acylamino (-NH-CO-R) in which R represents a linear, branched or cyclic, saturated or unsaturated C₁-C₁₀ alkyl group,
- carboxylic acid (-COOH),
- ester (-COOR) in which R represents a linear, branched or cyclic, saturated or unsaturated C₁-C₁₀ alkyl group,
as film-forming agent.

2. Use according to Claim 1, in which the compound is chosen from hyperbranched polymers, and in particular polyethyleneimine, comprising at least one group of formula (I).

3. Use according to either of the preceding claims, in which Y represents an oxygen atom.

4. Use according to one of the preceding claims, in which A represents a radical corresponding to one of the formulae (a) to (d) below:
(a) -CHR¹-CHR²-CHR³-
(b) -CHR'¹-CHR'²-CHR'³-CHR'⁴-
(c) 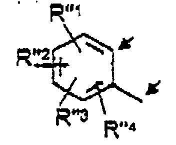
(d) -(CHR'''¹)ₖ-(CHR'''²)-CH(CO₂H)-NH-
in which
* R¹, R², R³, R'¹, R'², R'³ and R'⁴, R'''¹ and R'''², which may be identical or different, represent: a hydrogen atom; a linear, branched or cyclic, saturated or unsaturated C₁-C₆ alkyl radical; an amino (-NH₂) radical; a carboxylic acid (-COOH) radical; a C₁-C₁₀ alkylamino radical; a C₁-C₁₀ acylamino radical;
* R"¹, R"², R"³ and R"⁴, which may be identical or different, represent: a hydrogen atom; a linear or branched, saturated or unsaturated C₁-C₄ alkyl radical, the arrows indicating the positions of the substitutions;
* k is an integer, preferably 0 or 1.

5. Use according to one of the preceding claims, in which A is chosen from the following groups:
-CH₂-CH(CO₂H)-NH- ; -(CH₂)₂-(CH₃CONH)CH-; -(CH₂)₃- and -CH₂-CH(NH-CO-CH₃)-.

6. Use according to one of the preceding claims, in a cosmetic or pharmaceutical composition which also comprises a cosmetically or pharmaceutically acceptable medium.

7. Film-forming composition comprising at least one compound chosen from hyperbranched polymers and dendrimers, comprising at least one group of formula (I), as defined in one of Claims 1 to 5.

8. Composition according to Claim 7, which can be obtained by oxidation of a composition comprising at least one polymer chosen from hyperbranched polymers and dendrimers comprising at least one group of formula (II) : 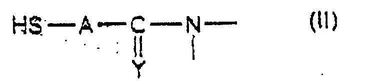 in which:
* Y represents an oxygen atom or an NH group, and
* A represents a linear, branched or cyclic, saturated or unsaturated C₁-C₁₂ alkanediyl group, this group optionally being interrupted with one or more hetero atoms and/or substituted with a function chosen from:
- amino (-NH₂),
- acylamino (-NH-COR) in which R represents a linear, branched or cyclic, saturated or unsaturated C₁-C₁₀ alkyl group,
- carboxylic acid (-COOH),
- ester (-COOR) in which R represents a linear, branched or cyclic, saturated or unsaturated C₁-C₁₀ alkyl group.

9. Composition according to either of Claims 7 and 8, in which the oxidation is carried out in air or using a common oxidizing agent such as hydrogen peroxide.

10. Composition according to one of Claims 7 to 9, in which the oxidation is carried out in the presence of water, for example in aqueous or aqueous-alcoholic medium.

11. Composition according to Claim 10, in which water-soluble or water-insoluble additives are incorporated, before oxidation, into the aqueous or aqueous-alcoholic medium.

12. Composition according to Claim 11, in which the said additives are chosen from water-soluble dyes such as Rhodamine; water-soluble cosmetic or pharmaceutical active agents; water-insoluble products which have in particular optical properties such as phosphorescence or fluorescence; pigments; fillers; sunscreens; water-insoluble cosmetic or pharmaceutical active agents.

13. Composition according to one of Claims 7 to 12, which is in the form of a cosmetic or pharmaceutical composition also comprising a cosmetically or pharmaceutically acceptable medium.

14. Composition according to Claim 13, which is in the form of lotions, milks or creams for skincare or haircare; make-up-removing creams, lotions or milks; foundation bases; antisun or after-sun lotions, milks or creams; artificial tanning lotions, milks or creams; shaving creams or foams; aftershave lotions; body hygiene compositions such as deodorant sticks or creams; shampoos; hair products for maintaining the hairstyle or for shaping the hair such as styling gels; hair-colouring products; lipsticks; mascaras or eyeliners which may be for treatment purposes; nail varnishes or nailcare products.

15. Use of at least one polymer chosen from hyperbranched polymers and dendrimers comprising at least one group of formula (II): 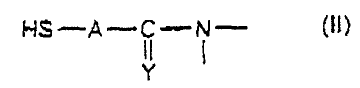 in which Y and A have the meanings defined above, for the preparation of a film-forming composition comprising at least one compound chosen from hyperbranched polymers and dendrimers comprising at least one group of formula (I), or of a composition according to one of Claims 7 to 14.

16. Process for obtaining a film on a support, in which a composition according to one of Claims 7 to 14 is applied to the said support.

17. Process for obtaining a film on a support, in which:
- a composition comprising a polymer chosen from hyperbranched polymers and dendrimers comprising at least one group of formula (II) is applied to the said support, and
- the said composition is oxidized during or after its application to the said support.

18. Process according to either of Claims 16 and 17, in which the support is chosen from the skin, mucous membranes, semi-mucous membranes; the nails and the hair.
